# EUROPÄISCHE PATENTSCHRIFT

(11) **EP 0 906 753 B1**
(45) Veröffentlichungstag und Bekanntmachung des Hinweises auf die Patenterteilung: **04.06.2003**
(21) Anmeldenummer: 97116053.6
(22) Anmeldetag: 16.09.1997
(51) Int. Cl.: A61K 9/00

(54) **Brausezubereitung und Verfahren zu ihrer Herstellung**
Effervescent preparation and process for producing the same
Composition effervescente et son procédé de préparation

(43) Veröffentlichungstag der Anmeldung: 07.04.1999
(73) Patentinhaber: Dr. Gergely & Co., 1053 Wien (AT)
(72) Erfinder: Gergely, Gerhard, Dr., A-1053 Wien (AT); Gergely, Irmgard, A-1053 Wien (AT)
(74) Vertreter: Büchel, Kurt F.

(56) Entgegenhaltungen:
- EP-A- 0 203 768
- EP-A- 0 396 972
- EP-A- 0 418 564
- EP-A- 0 670 160

## Beschreibung

Die Erfindung betrifft eine Brausezubereitung nach dem Oberbegriff des Anspruches 1 und ein Verfahren zu ihrer Herstellung nach dem Oberbegriff des Anspruches 10.

In Abhängigkeit vom Verhalten des jeweils gewünschten Wirkstoffes in der Brausezubereitung wurden schon die verschiedensten Mischungen oder Massnahmen vorgeschlagen. Besonders kleine und insbesondere hydrophobe unlösliche Wirkstoffteilchen bereiten während des Brausens der Zubereitung in Wasser Probleme, weil sie von den Gasblasen an die Flüssigkeitsoberfläche mitgerissen werden und in dem sich dort bildenden Schaum hängen bleiben statt sich in der Flüssigkeit aufzulösen oder zu suspendieren.

Nun wurde bereits in der EP-B-768.868 vorgeschlagen, eine Kombination von zwei Emulgatoren, oder eines Emulgators mit einem Tensid zu verwenden, um diese Probleme zu beseitigen. Dies gelingt auch für manche Wirkstoffe wenigstens teilweise. Für solche mit besonders kleinen Korngrössen und besonders starker Hydrophobie, sowie gegebenenfalls noch einer sperrigen Kornform sind diese Massnahmen aber nicht ausreichend.

Die Erfindung hat sich daher die Aufgabe gestellt, eine Brausezubereitung und ein Verfahren zu ihrer Herstellung zu schaffen, mit Hilfe derer die genannten Probleme auch für Wirkstoffteilchen mit besonders kleinen Korngrössen und besonders starker Hydrophobie, sowie gegebenenfalls noch einer sperrigen Kornform, dazu gehört insbesondere z.B. Adamantin-Aminosulfat (kurz Amantadinsulfat genannt), gelöst werden können.

Amantadinsulfat wird in der Anti-Parkinson-Therapie eingesetzt und kann auch als antivirales Mittel für Influenza-Typ-A-Bekämpfung Anwendung finden. Amantadinsulfat wird derzeit hauptsächlich in Form von Tabletten verabreicht. Da bei vielen Parkinsonpatienten Schluckbeschwerden auftreten und sie Mühe haben, Tabletten zu schlucken, wurde angestrebt, das Amantadinsulfat in eine lösliche Tablette oder in eine Brausetablette zu inkorporieren, um die Verabreichung für die Patienten zu erleichtern.

Das größte Problem stellte die extrem hydrophobe Eigenschaft des Wirkstoffes dar, der eine äußerst schlechte Benetzbarkeit zeigt, wodurch es beim Zumischen des Wirkstoffes zu einer Brausebasis und beim Verpressen zu Tabletten bei der Auflösung der Brausetablette in Wasser zu einer sehr starken Schaumbildung kommt, in dem der Wirkstoff an der Oberfläche festgehalten wird.

Amantadinsulfat ist zwar in sauren Lösungen löslich, jedoch ist die erforderliche Zeit, die es benötigt, um sich in einer Brausetösung von einem pH von 4 zu lösen, wesentlich länger als die Auflöszeit der Brausetablette beträgt. Deshalb wird der Wirkstoff beim brausenden Auflösen der Tablette in Wasser mit den CO₂-Bläschen an die Oberfläche getragen und wird dort im Schaum festgehalten, sodaß keine gleichförmige Suspension entsteht und damit die Möglichkeit gegeben wäre, daß sich das Amantadinsulfat im Laufe der Zeit lösen kann.

Auch das Zufügen zulässiger Emulgatoren, wie von Polysorbaten oder Lecithin-Emulgatoren; wie auch von Benetzungsmitteln, wie Dioctylsulfosuccinat, Natriumlaurylsulfat und anderen, konnten nicht helfen, dieses Phänomen wesentlich zu verbessern und den Wirkstoff in einer Brauselösung zu suspendieren bzw. das Schaumverhalten zu reduzieren, ebenso wie die Verwendung von Anti-Schaummitteln nicht zu dem gewünschten Erfolg führte.

Um die schlechten Suspensionseigenschaften des Amantadinsulfats zu verbessern, wurde entsprechend der EP-A1-241.469 versucht, den Wirkstoff mit Benetzungsmittel, wie Docusat-Natrium oder Natriumlaurylsulfat mit und ohne Bindemittel zu behandeln. Dieses Vorgehen war aber leider nicht zielführend, und zwar weder durch direktes Aufbringen des Tensids auf den Wirkstoff, noch in Kombination mit einem Hilfsstoff.

Außerdem kam es dabei partiell zur Granulierung von Wirkstoffteilchen und somit zu deren Vergrößerung, wobei die gröberen Teilchen zu Boden sinken und aufgrund der mangelnden Durchwirbelung einen Rückstand bilden, die feinen Teilchen aber an die Oberfläche steigen und dort zu einer starken Schaumbildung führen, die auch durch Zusatz eines Anti-Schaummittels nicht wesentlich reduziert werden konnte.

Auch die Verarbeitung des Amantadinsulfats entsprechend dem erwähnten EP-B-768.868 führte in diesem Falle nicht zu den angestrebten Auflösungseigenschaften, da bei der Anbindung des Wirkstoffes an einen Schwebstoff, z.B. Aerosil, der Schwebstoff teils mit dem Wirkstoff an die Oberfläche schwimmt, bzw. bei einem Füllstoff wie Mannitol oder Lactose ohne Schwebstoff bei der Beladung mit.dem Wirkstoff Partikel entstehen, die zu Boden sinken. Der Wirkstoff konnte nur durch Umschütten nach dem Auflösen der Brausetablette in Suspension gebracht werden.

Ziel der Erfindung war daher wie erwähnt, eine galenische Zubereitung für Amantadinsulfat in Form einer Brausetablette zu schaffen, die dem physikalischen Verhalten dieses Wirkstoffs Rechnung trägt und vor allem die Schaumbildung, sowie das Ansammeln des Wirkstoffes im Schaum verhindert. Die Lösung dieser Aufgabenstellung bzw. Probleme ergibt sich erstmalig überraschend durch die im Kennzeichen des Anspruches 1 beschriebenen Maßnahmen. Besondere Ausführungsformen und Weiterentwicklungen des Erfindungsgedankens sind in den Kennzeichen der abhängigen Ansprüche beschrieben.

Die Erfindung beruht darauf, den Wirkstoff in einer eigenen Phase unterzubringen, indem er mittels eines Bindemittels mit dem Salz einer eßbaren organischen Säure, wie z.B. Mononatriumcitrat, Trinatriumcitrat bzw. Mononatriumtartrat granuliert wird, wobei bevorzugt die sauren Salze der Zitronensäure und Weinsäure Verwendung finden.

Durch die Anbindung des Wirkstoffes an das Salz einer Säure kommt es beim Auflösen der Tablette zu einer langsamen Reaktion mit dem Bicarbonat der Brausebasis und einer nur geringen CO₂-Entwicklung, die dem Wirkstoff Zeit gibt, in der Lösung zu suspendieren ohne sofort an die Oberfläche geschleudert zu werden.

Diese Vorgehensweise hat sich als vorteilhafter erwiesen, als den Wirkstoff an die Säure selbst zu binden, da bei den leichtlöslichen Säuren, wie Zitronensäure und Weinsäure, bei der Auflösung der Tablette in Wasser eine zu heftige Reaktion mit den Alkalibestandteilen der Brausebasis stattfindet, wodurch der Wirkstoff zu schnell durch die Reaktion und die Auflösung der Zitronensäure aus der Phase freigesetzt wird. Dieses Phänomen zeigt sich auch dann, wenn man höhere Polyvinylpyrrolidon- (PVP-)Mengen verwendet, die überdies den Nachteil haben, eine noch erhöhte Schaumbildung zu bewirken. Beim Einsatz von schwerlöslichen Säuren, wie z. B. Fumarsäure oder Adipinsäure, ist die Reaktion hingegen wieder zu langsam, und die Teilchen sinken zu Boden, bevor sie gelöst werden können.

Die Wirkstoffphase mit dem Salz einer löslichen, eßbaren, organischen Säure wird zu einer Brausebasis zugemischt, die in Form einer einfachen Mischung der Brausekomponenten, aber auch in Form einer eigenen Phase vorliegen kann, in der die Brausebasis granuliert ist; vorzugsweise sind die Säurekristalle mittels einer Salzschicht aus einer Teilreaktion zwischen der Säure und dem Bikarbonat - mit den Bicarbonatteilchen abgedeckt bzw. überzogen. Die Brausebasis kann überdies mit Zusatzstoffen, sowie weiteren Füllstoffen, und gegebenenfalls mit Emulgatoren und Aromen versetzt werden.

Das Verhältnis von Amantadinsulfat zu dem Salz einer eßbaren organischen Säure kann zwischen 1:0,5 und 1:5, bevorzugt bei etwa 1:3, liegen, wobei die Relation auch vom angestrebten Tablettengewicht abhängig ist.

Die Granulierung von Amantadinsulfat mit dem Salz der organischen Säure erfolgt bevorzugt mit dem Bindemittel Polyvinylpyrrolidon, das in einem Verhältnis zum Amantadinsulfat von 1:0,05 bis 1:0,2 eingesetzt wird, oder auch mit Zein.

Um den PVP-Film elastischer zu gestalten und die Benetzung zu verbessern, kann Glycerin zur Wirkstoffphase zugesetzt werden. Eine weitere Verbesserung des Auflöseverhaltens des Wirkstoffes konnte durch Einbringen eines neutralen Hilfs- oder Füllstoffes in die Wirkstoffphase erreicht werden, z.B. eines Zuckeralkohols, wie Mannitol oder Sorbitol, aber auch das Hydrokolloid Maltodextrin. Das Verhältnis solcher neutraler - vorzugsweise löslicher - Hilfs- bzw. Füllstoffe zum Wirkstoffanteil in der Phase kann von 1:0,5 bis 1:4 betragen.

Es hat sich weiters als vorteilhaft erwiesen, in die Wirkstoffphase auch ein Alkalibicarbonat in pulverisierter Form einzubringen, um damit in den Granulatteilchen der Wirkstoffphase während des Auflösens der Tablette durch die Reaktion mit dem sauren Salz bei Wasserzutritt einen schnelleren Zerfall zu erreichen.

Die Herstellung des Wirkstoffgranulates erfolgt zweckmässigerweise folgendermaßen: der Wirkstoff wie z.B. Amantadinsulfat (Korngröße 5-15 µm) wird mit Mononatriumtartrat und/oder Mononatriumcitrat, und bevorzugt auch noch Natriumhydrogencarbonat gemischt; der Mischung kann anschließend Sorbitol, Mannitol oder Maltodextrin zugemischt werden, worauf sie mit einer Lösung aus PVP in 70 %igem Alkohol, dem bevorzugt noch etwas Glycerin zugesetzt wurde, granuliert wird. Das Granulat wird getrocknet und auf 0,8 - 2 mm vermahlen, mit den restlichen Bestandteilen der vorgesehenen Brausezubereitung, d.h. ausser den Komponenten der Brausebasis auch pharmazeutisch üblichen bzw. zulässigen Zusatz- und Hilfsstoffen, vermischt und gegebenenfalls zu Tabletten verpresst.

In der Brausebasis können alle gängigen Brausebestandteile Verwendung finden, wobei bevorzugt die Säurekomponente aus Zitronensäure, Weinsäure, Äpfelsäure bzw. deren Salzen, wie Mononatriumcitrat oder Mononatriumtartrat, besteht. Der Basenanteil der Brausebasis besteht zweckmäßigerweise aus CO₂-abspaltenden Alkalibicarbonaten oder Carbonaten, wie Natriumund/oder Kaliumhydrogencarbonaten oder Carbonaten, sowie aus Erdalkalicarbonaten, wobei es in letzterem Fall nicht empfehlenswert ist, z.B. Calciumcarbonat bzw. Magnesiumcarbonat ausschließlich als Basenanteil zu verwenden, da damit die Schaumbildung begünstigt wird.

Als Zusatzstoffe und Hilfsstoffe können insbesondere Süßstoffe, wie Zucker, Cyclamat, Sacharin, Aspartam, Acesulfam, sowie Farbstoffe, Geschmacksstoffe oder andere galenische Zusatzstoffe, wie Zuckeralkohole, z.B. Mannitol und Sorbitol, wie auch Maltodextrin, verwendet werden.

Um die Auflösungseigenschaften noch zu verbessern, kann zu der Mischung des Brausegranulates und der Wirkstoffphase noch ein Anti-Schaummittel in Form einer Phase, bei der das Anti-Schaummittel auf einen neutralen Hilfs- bzw. Füllstoff aufgezogen wird, sowie eine Emulgatorphase, bevorzugt bestehend aus einem hydrierten Rhizinusöl auf einem Trägerstoff, zugefügt werden. Zur Herstellung dieser Phasen werden der Emulgator bzw. das Anti-Schaummittel mittels eines Lösungsmittels oder als wässrige Suspension auf einen Füllstoff aufgezogen, um innerhalb der Tablette eine möglichst ubiquitäre Verteilung zu erreichen.

Um aber diese Zubereitung in der Brauselösung optimal zu suspendieren und zu verhindern, daß der Wirkstoff sich beim Auflösen der Tablette an der Oberfläche sammelt, ist es empfehlenswert, in die Gesamt-Rezeptur eine oberflächenaktive Substanz einzubringen. Empfehlenswert sind Phospholipide, z.B. Lecithine, Metharin, Epikuron sowie auch Polysorbate, DK-Ester oder Zuckerester. Als besonders geeignet hat sich Polyethylenglycol PEG 40, ein hydriertes Rhizinusöl, gezeigt. Bevorzugt wird ein Emulgatorgranulat getrennt von der Brausebasis und vom Wirkstoffgranulat hergestellt, wobei als Träger für den Emulgator fast alle für eine Brausetablette geeigneten Füllstoffe, wie höhere Alkohole, z.B. Mannitol, Sorbitol oder Lactose, ebenso Hydrokolloide, wie Maltodextrin oder Dextrin, gegebenenfalls aber auch Stärke in Frage kommen. Empfehlenswert ist ein Verhältnis von 1 Teil Emulgator auf 100 Teile Träger. Die Dosierung des Emulgators beträgt - auf den Wirkstoff bezogen - etwa 0,5 bis 2 Gewichtsteile.

Die oberflächenaktiven Substanzen können aber auch einzeln oder auch in Mischungen in die Amantadinsulfat-Phase eingebracht werden. Dazu wird eine Lösung hergestellt, die auf 100 Gewichtsteile Amantadinsulfat 0,15 Gewichtteile Docusat-Natrium, 0,48 Gewichtsteile Tween® 80 sowie 0,12 Gewichtteile Emulgin C 700 (d. i. eine Mischung nicht ionogener Emutgatoren, bestehend aus Glycerinester von Fettsäuren) in 40%igem Alkohol mit oder ohne Bindemittel enthält. Die Amantadin-Phase wird dann mit dieser Lösung granuliert. Bei Zusatz eines Bindemittels können 10 Gewichtteile des Bindemittels, wie z.B. PVP, mit eingebracht werden.

Eine erfindungsgemäß hergestellte pharmazeutische Brausezubereitung mit Amantadinsulfat zeichnet sich durch eine schnelle Auflösung in Wasser und ein gutes Auflöseverhalten aus. Die Erfindung wird im Folgenden anhand einiger Beispiele erläutert.

### Beispiel 1

### (Negativbeispiel, Stand der Technik; Amantadinsulfat-Phase mit Zitronensäure):

100 Gewichtsteile unbehandeltes Amantadinsulfat, 200 Gewichtsteile Zitronensäure pulvis und 50 Gewichtsteile Natriumbicarbonat werden mit 22 Gewichtsteilen einer Lösung bestehend aus 10 Gewichtsteilen Propylenglycol, 0,5 Gewichtsteilen Tween® und 30 Gewichtsteilen PVP granuliert. Die Masse wird getrocknet und anschließend auf 0.4 mm gesiebt.

Diese Phase wird dann mit einer Brausebasis vermischt, die folgende Zusammensetzung hat: 150 Gewichtsteile Zitronensäure kristallin und 350 Gewichtsteile Mononatriumcitrat werden auf 60°C erwärmt und mit 4 Gewichtsteilen einer 50%igen Zitronensäure-Lösung in Wasser befeuchtet; anschliessend werden 55 Gewichtsteile Natriumbicarbonat anreagieren lassen. Abschliessend werden - vor dem Trocknen - 50 Gewichtsteile Natriumcarbonat zugefügt; das Produkt wird unter langsamem Rühren mittels Vakuum getrocknet und abschliessend auf 1,5 mm gesiebt. Zu der Mischung dieser Brausebasis mit der Amantadinsulfat-Phase fügt man noch 20 Gewichtsteile Aspartam sowie 6 Gewichtsteile Aroma hinzu, und weiters noch 100 Gewichtsteile einer Antischaummittel-Phase bestehend aus 100 Gewichtsteilen Mannitol und 0,2 Gewichtsteilen Simethicon. Die fertige Mischung wird zu Tabletten verpresst. Das Produkt zeigt eine ziemlich dichte Schaumbildung; ca. 28 Gew.% des Wirkstoffes werden in der Schaumschichte bzw. an der Grenzfläche zum Schaum festgehalten, und 8-9 Gew.% sinken zu Boden.

### Beispiel 2

### (Negativbeispiel, Amantadinsulfat auf einem Lactose-Träger)

100 Gewichtsteile Amantadinsulfat und 370 Gewichtsteile Lactose D80 werden homogen vermischt und auf 50°C erwärmt; anschliessend wird die Mischung mit einer Lösung aus 9 Gewichtsteilen PVP K25 und 0,5 Gewichtsteile Tween® 80 in 20 Gewichtsteilen 60%igem Ethanol granuliert. Das Produkt wird mittels Vakuum getrocknet und abschliessend auf 0,3 mm gesiebt.

Diese Phase wird mit der Brausebasis von Beispiel 4 vermischt und zu Tabletten mit 1,4 g verpresst. Bei der Auflösung entsteht viel Schaum, und es zeigt sich eine schlechte Suspension des Wirkstoffs; die Lösung bleibt trüb.

### Beispiel 3

### (Negativbeispiel mit Schwebstoff analog der EP-A1-241.469)

100 Gewichtsteile Amantadinsulfat werden mit 300 Gewichtsteilen Sorbitol Pulver sowie 20 Gewichtsteilen Aerosil® vermischt und auf 45°C aufgeheizt. Diese Mischung wird mit einer Lösung auf 9 Gewichtsteilen PVP, 0,2 Gewichtsteilen Docusat-Natrium sowie 0,5 Gewichtsteilen Tween® 80 in 70 Gewichtsteilen 80%igem Alkohol granuliert. Das Granulat wird anschiessend mittels Vakuum getrocknet, die getrocknete Masse wird auf 0,3 mm gesiebt.

Diese Amantadinsulfat-Phase, vermischt mit der Brausebasis aus Beispiel 4, ergibt beim Auflösen in Wasser noch eine gewisse Trübung und eine unschöne Schaumbildung, wobei sich über 30 Gew.% des Wirkstoffs teilweise im ziemlich grossblasigen, über mehrere Minuten stabilen Schaum, teilweise an der Grenzfläche zum Schaum ansammeln.

### Beispiel 4

### (Amantadinsulfat-Phase mit Mononatriumcitrat)

100 Gewichtsteile Amantadinsulfat, 200 Gewichtsteile Mononatriumcitrat und 100 Gewichtsteile Sorbitol Pulver sowie 20 Gewichtsteile Natriumhydrogencarbonat Pulver werden homogen vermischt. Daneben wird eine Suspension hergestellt, indem in eine Lösung aus 10 Gewichtsteilen PVP und 5 Gewichtsteilen Glycerin in ca. 25 Gewichtsteilen 70%igem Ethanol 20 Gewichtsteile Natriumhydrogencarbonat Pulver suspendiert wurden. Mit dieser Suspension wird die eingangs genannte Pulvermischung granuliert. Das entstehende Granulat wird mittels Vakuum getrocknet und abschließend durch ein 1,0 mm Sieb gesiebt.

Diese Phase wird mit einer Brausebasis vermischt, die aus 565 Gewichtsteilen Zitronensäure, 275 Gewichtsteilen Natriumhydrogencarbonat, 7,5 Gewichtsteilen Natriumcarbonat, sowie 20 Gewichtsteilen Aspartam mittels einer Trinatriumcitrat-Lösung granuliert, anschließend getrocknet und auf 1,6 mm gesiebt wurde. Zur Mischung der Brausebasis und der Amantadinsulfat-Phase werden weiters neutrale Hilfs- bzw. Füllstoffe, nämlich 119 Gewichtsteile Sorbitol, 100 Gewichtsteile Lactose und 6 Gewichtsteile Aroma hinzugefügt, sowie auch 100 Gewichtsteile einer Emulgator-Phase bestehend aus 1 Teil Polyethylenglycol PEG 40 auf 100 Teilen Träger sowie 100 Gewichtsteile einer Antischaummittel-Phase, bestehend aus 0,1 Gewichtsteilen Simethicon auf 100 Gewichtsteilen Mannitol.

Die fertige Mischung wird zu Tabletten mit einem Tablettengewicht von 1,6 g verpreßt, zeigt eine Auflösezeit von ca. 80 - 100 Sekunden und weist nur eine geringe Schaumbildung auf.

### Beispiel 5

### (Zitronensäure und Mononatriumcitrat in der Brausebasis)

Man geht analog Beispiel 4 vor, nur wird statt der Brausebasis mit Zitronensäure eine Brausebasis verwendet, die eine Mischung aus Zitronensäure und Mononatriumcitrat enthält, und zwar mit folgender Zusammensetzung: 200 Gewichtsteile Zitronensäure fein kristallin, 100 Gewichtsteile Zitronensäure pulvis und 350 Gewichtsteile Mononatriumcitrat werden vermischt und in einem beheizbaren Vakuumgranulator auf 60°C aufgeheizt. Diese Mischung wird mit einer Lösung aus Zitronensäure:Wasser 1:1 benetzt und anschließend 55 Gewichtsteile Natriumhydrogencarbonat zugefügt, das man definiert anragieren läßt und zum Abschluß werden 50 Gewichtsteile Natriumcarbonat zugefügt. Nach der Granulierung erfolgt die Trocknung bevorzugt mit Vakuum und das Granulat wird nach abschließendem Trocknen 1,5 mm gesiebt.

### Beispiel 6

### (Amantadinsulfat-Phase mit Mononatriumtartrat)

In einem beheizbaren Vakuumgranulator werden 100 Gewichtsteile Amantadinsulfat. 300 Gewichtsteile Mononatriumtartrat.H₂O sowie 20 Gewichtsteile Natriumhydrogencarbonat homogen vermischt und auf 50°C aufgeheizt. Anschließend werden 100 Gewichtsteile Sorbitol homogen zugemischt. Die resultierende Pulvermischung wird mit einer Lösung von 10 Gewichtsteilen Polyvinylpyrrolidon und 5.7 Gewichtsteilen Glycerin in 300 Gewichtsteilen 70%igem Ethanol, in der 20 Gewichtsteile Natriumhydrogencarbonat Pulver suspendiert wurden, granuliert. Das Granulat wird bei 50°C unter langsamem Rühren mittels Vakuum getrocknet und anschließend auf 1,5 mm gesiebt.

Die resultieende Amantadinsulfat-Phase wird mit einer Zitronensäure-Brausebasis vermischt, die aus folgenden Bestandteilen hergestellt wird: 415 Gewichtsteile Zitronensäure Feingrieß, 100 Gewichtsteile Zitronensäure pulvis werden in einem Vakuumgranulator auf 60°C aufgeheizt, anschließend mit einer Mononatriumcitrat-Lösung granuliert, sodann werden 220 Gewichtsteile Natriumbicarbonat und 16,5 Gewichtsteile Aspartam zugefügt. Man lässt unter kontrolliertem Vakuum reagieren, fügt vor dem Trocknen 27 Gewichtsteile Natriumcarbonat hinzu und trocknet dann mittels Vakuum bis 15 mbar. Die getrocknete Brausebasis wird durch ein 2 mm-Sieb ausgesiebt.

Diese Brausebasis wird mit der Amantadinsulfat-Phase sowie mit 100 Gewichtsteilen einer Antischaummittel-Phase, -Phase, bestehend aus 0,1 Gewichtsteilen Simethicon auf 100 Gewichtsteile Mannitol sowie einer Mannitol-Emulgator-Phase, bestehend aus 100 Gewichtsteilen Mannitol und 1 Gewichtsteil Polyethylenglycol PEG 40 (ein hydriertes Rhizinusöl) sowie weiters mit 10 Gewichtsteilen Aroma vermischt. Das Produkt wird zu Tabletten von 1,6 g verpreßt, die eine Auflöszeit von 85 Sekunden zeigen, fast keine Schaumbildung mehr aufweisen und eine gute Suspension sowie auch eine teilweise Lösung des Wirkstoffes Amantadinsulfat gewährleisten. Nur mehr 9 Gew. % des Wirkstoffs finden sich in der Schaumschicht bzw. an der Grenzfläche, werden aber bei auch nur geringem Umschütteln in der Lösung sehr schön suspendiert. Ein Rückstand ist nicht mehr bestimmbar. Dies mag darauf zurückzuführen sein, dass die Weinsäure ein starker Komplexbildner ist, wodurch sich zusätzlich die Beseitigung des eingangs erwähnten Problems wesentlich verbessern lässt. Bei Bedarf können in der Mischung vor dem Verpressen noch Füllstoffe, wie Sorbitol, Mannitol, Dextrin, etc. möglichst homogen verteilt werden.

### Beispiel 7

### (Naproxen-Phase mit Mononatriumtartrat oder Mononatriumcitrat):

150 Gewichtsteile Naproxen, 750 Gewichtsteile Mononatriumtartrat oder Mononatriumcitrat, sowie 250 Gewichtteile Sorbitol werden unter Mischen auf 60°C erwärmt. Man fügt dann 50 Gewichtsteile Natriumhydrogencarbonat zu und granuliert mit einer Lösung aus 25 Gewichtsteilen PVP in 87 Gewichtteilen 70%igen Ethanols, in der 50 Gewichtteile Natriumhydrogencarbonat suspendiert sind. Das Produkt wird mittelsVakuum getrocknet und anschliessend auf 1,0 mm gesiebt.

1375 Gewichtteile dieser Wirkstoff-Phase werden mit 1944 Gewichtteilen Brausebasis nach Beispiel 4, sowie mit 250 Gewichtteilen Antischaummittel-Phase (0,1 Gewichtteil Simethicon auf 100 Gewichtteile Mannitol), und 250 Gewichtteilen einer 1%igen Emulgator-Phase (100 Gewichtteile Mannitol mit 1 Gewichtteil Polyethylenglycol PEG 40), sowie 25 Gewichtteilen Aroma, 155 Gewichtteilen Sorbitol und 125 Gewichtteilen Lactose vermischt und zu Tabletten von 4,1 g verpresst.

Beide Produkte zeigen eine geringe Schaumbildung und ein gutes Suspensionsverhalten.

## Patentansprüche

1. Brausezubereitung zur Auflösung in Wasser, mit einer Brausebasis aus sauren und gasabspaltenden Bestandteilen und wenigstens einem pharmazeutischen Wirkstoff, sowie gegebenenfalls weiteren Hilfsstoffen wie z. B. Aromen, Süssstoffen, Füllmitteln oder dergleichen, **dadurch gekennzeichnet, dass** der Wirkstoff in Granulatteilchen ("Wirkstoff-Phase") mit einem pharmazeutisch zulässigen Bindemittel an wenigstens ein Salz einer essbaren organischen Säure gebunden vorliegt, welche Wirkstoff-Phase mit der Brausebasis und dem weiteren pharmazeutisch zulässigen Hilfsstoffen vermischt sowie gegebenenfalls zu Tabletten verpresst wird.

2. Brausezubereitung nach Anspruch 1, **dadurch gekennzeichnet, dass** der Wirkstoff in Granulatteilchen (_{"}Wirkstoff-Phase") weiters mit einem neutralen Hilfs- bzw. Füllstoff vorliegt.

3. Brausezubereitung nach einem der vorhergehenden Ansprüche, **dadurch gekennzeichnet, dass** der Wirkstoff in einer Korngrösse unter 100, vorzugsweise unter 50, insbesondere unter 20 µm vorliegt.

4. Brausezubereitung nach einem der vorhergehendenAnsprüche, **dadurch gekennzeichnet, dass** wenigstens ein Teil des Salzes als ein saures Salz, insbesondere etwa - auf Wirkstoff-Phase bezogen - 35 bis 55 Gewichtsprozent Mononatriumcitrat und/oder etwa 40 bis 65 Gewichtsprozent Mononatriumtartrat, bzw. aliquote Mischungen der Beiden vorliegt.

5. Brausezubereitung nach einem der vorhergehenden Ansprüche, **dadurch gekennzeichnet, dass** das Bindemittel der Wirkstoff-Phase - auf 100 Gewichtsteile Wirkstoff bezogen - etwa 4 bis etwa 13, vorzugsweise 7 bis 12 Gewichtsteile Polyvinylpyrrolidon ist.

6. Brausezubereitung nach einem der vorhergehenden Ansprüche, **dadurch gekennzeichnet, dass** die sauren Bestandteile der Brausebasis wenigstens eine feste, essbare, organische Säure in Mischung mit wenigstens einem sauren Salz einer solchen Säure sind.

7. Brausezubereitung nach einem der vorhergehenden Ansprüche, **dadurch gekennzeichnet, dass** die Wirkstoff-Phase einen Teil der gasabspaltenden Bestandteile in der Zubereitung, vorzugsweise etwa 5 bis etwa 15, besonders vorzugsweise 8 bis 12 Gewichtsprozent der Gesamtmenge gasabspaltender Bestandteile in der Zubereitung, enthält.

8. Brausezubereitung nach einem der vorhergehenden Ansprüche, **dadurch gekennzeichnet, dass** die Wirkstoff-Phase etwa 15 bis etwa 25, vorzugsweise 18 bis 22 Gewichtsprozent des neutralen Hilfs- bzw. Füllstoffes, insbesondere eine der Substanzen Sorbitol, Mannitol und/oder Maltodextrin, enthält.

9. Brausezubereitung nach einem der vorhergehenden Ansprüche, **dadurch gekennzeichnet, dass** sie aus wenigstens je einer voneinander getrennt vorliegenden Brausebasis und Wirkstoff-Phase, sowie vorzugsweise auch einer Emulgator- und/oder einer Antischaummittel-Phase besteht.

10. Verfahren zur Herstellung einer Brausezubereitung nach einem der vorhergehenden Ansprüche, **dadurch gekennzeichnet, dass** zunächst getrennt von einander eine Wirkstoff-Phase und eine Brausebasis hergestellt werden, wobei zur Herstellung der Wirkstoff-Phase der Wirkstoff mit einem - vorzugsweise sauren - Salz einer essbaren organischen Säure, sowie gegebenenfalls wenigstens einem pharmazeutisch zulässigen Bindemittel und/oder wenigstens einem pharmazeutisch zulässigen, neutralen Hilfs- bzw. Füllstoff und/oder einem Teil der gasabspaltenden Bestandteile gemischt und mit einer ethanolischen Lösung eines Bindemittels, in dem als gasabspaltender Bestandteil Natriumbicarbonat suspendiert wurde, granuliert, getrocknet und auf die gewünschte Korngrösse vermahlen wird, während die Brausebasis aus einer festen, essbaren, organischen Säure, sowie gegebenenfalls wenigstens einem - vorzugsweise sauren - Salz einer solchen Säure in Mischung mit Alkali- und/oder Erdalkalicarbonaten oder -bikarbonaten, vorzugsweise unter Zusatz eines Süssstoffes, besteht und granuliert, getrocknet und auf die gewünschte Korngrösse gemahlen wird, worauf schliesslich die beiden Phasen - vorzugsweise unter Zusatz weiterer, insbesondere neutraler, Hilfs- bzw. Füllstoffe - vermischt und gegebenenfalls zu Tabletten verpresst werden.

11. Verfahren nach Anspruch 10, **dadurch gekennzeichnet, dass** ausserdem wenigstens eine Emulgator- und/oder Antischaummitelphase getrennt voneinander hergestellt werden, indem auf 100 Gewichtsteile eines Trägerstoffes, wie z.B. Mannitol oder Sorbitol, 0.02 bis 1.0, vorzugsweise etwa 0.1 Gewichtsteile wenigstens eines Antischaummittels, bzw. 0.2 bis 2.0, vorzugsweise etwa 1.0 Gewichtsteile wenigstens eines Emulgators und/oder Tensids aufgezogen werden, worauf die so hergestellte Emulgator- und/oder Antischaummitelphase der Mischung aus Brausebasis und Wirkstoff-Phase zugemischt werden.

## Claims

1. Effervescent preparation for dissolution in water, comprising an effervescent base of acidic and gas-eliminating components and at least one pharmaceutical active substance, and optionally further excipients, such as, for example, flavours, sweeteners, fillers or the like, **characterized in that** the active substance is present bound to at least one salt of an edible organic acid with a pharmaceutically permitted binder in granular particles ("active substance phase"), which active substance phase is mixed with the effervescent base and the further pharmaceutically permitted excipients and is optionally pressed to give tablets.

2. Effervescent preparation according to Claim 1, **characterized in that** the active substance is present in granular particles . ("active substance phase") furthermore with a neutral excipient or filler.

3. Effervescent preparation according to either of the preceding Claims, **characterized in that** the active substance is present in a particle size of less than 100, preferably less than 50, in particular less than 20, µm.

4. Effervescent preparation according to any of the preceding Claims, **characterized in that** at least a part of the salt is present in the form of an acidic salt, in particular about - based on active substance phase - 35 to 55 percent by weight of monosodium citrate and/or about 40 to 65 percent by weight of monosodium tartrate, or aliquot mixtures of the two.

5. Effervescent preparation according to any of the preceding Claims, **characterized in that** the binder of the active substance phase - based on 100 parts by weight of active substance - comprises about 4 to about 13, preferably 7 to 12, parts by weight of polyvinylpyrrolidone.

6. Effervescent preparation according to any of the preceding Claims, **characterized in that** the acidic components of the effervescent base are at least one solid, edible, organic acid mixed with at least one acidic salt of such an acid.

7. Effervescent preparation according to any of the preceding Claims, **characterized in that** the active substance phase contains a part of the gas-eliminating components in the preparation, preferably about 5 to about 15, particularly preferably 8 to 12, percent by weight of the total amount of gas-eliminating components in the preparation.

8. Effervescent preparation according to any of the preceding Claims, **characterized in that** the active substance phase contains about 15 to about 25, preferably 18 to 22, percent by weight of the neutral excipient or filler, in particular one of the substances sorbitol, mannitol and/or maltodextrin.

9. Effervescent preparation according to any of the preceding Claims, **characterized in that** it consists of at least one each of effervescent base and active substance phase present separately from one another, and preferably also an emulsifier phase and/or an antifoam phase.

10. Process for producing an effervescent preparation according to any of the preceding Claims, **characterized in that** first an active substance phase and an effervescent base are prepared separately from one another, wherein, for the preparation of the active substance phase, the active substance is mixed with a - preferably acidic - salt of an edible organic acid, and optionally at least one pharmaceutically permitted binder and/or at least one pharmaceutically permitted, neutral excipient or filler and/or a part of the gas-eliminating components, and granulated with an ethanolic solution of a binder in which sodium bicarbonate was suspended as a gas-eliminating component, dried and milled to the desired particle size, while the effervescent base consists of a solid, edible, organic acid and optionally at least one - preferably acidic - salt of such an acid mixed with alkali metal and/or alkaline earth metal carbonates or bicarbonates, preferably with the addition of a sweetener, and is granulated, dried and milled to the desired particle size, whereupon finally the two phases are mixed - preferably with the addition of further, in particular neutral, excipients or fillers - and optionally pressed to give tablets.

11. Process according to Claim 10, **characterized in that** at least one emulsifier phase and/or antifoam phase are also prepared separately from one another by applying 0.02 to 1.0, preferably about 0.1, part by weight of at least one antifoam or 0.2 to 2.0 parts by weight, preferably about 1.0 part by weight, of at least one emulsifier and/or surfactant to 100 parts by weight of a vehicle, such as, for example, mannitol or sorbitol, whereupon the emulsifier phase and/or antifoam phase thus prepared are mixed with the mixture of effervescent base and active substance phase.

## Revendications

1. Composition effervescente pour dissolution dans de l'eau, avec une base effervescente comprenant des composantes acides et des composantes produisant un gaz et au moins une substance pharmaceutiquement active, ainsi que, le cas échéant, d'autres adjuvants tels que, par exemple, des arômes, des édulcorants, des excipients ou similaire, **caractérisée en ce que** la substance active est présente dans des particules granulaires (« phase de substance active »), liée par un agent liant pharmaceutiquement acceptable à au moins un sel d'un acide organique alimentaire, cette phase de substance active étant ensuite mélangée avec la base effervescente et, le cas échéant, avec d'autres adjuvants pharmaceutiquement acceptables, puis, le cas échéant, comprimée.

2. Composition effervescente selon la revendication 1, **caractérisée en ce que** la substance active est présente dans les particules granulaires (« phase de substance active ») avec, en outre, un adjuvant ou un excipient neutre.

3. Composition effervescente selon l'une des revendications précédentes, **caractérisée en ce que** la substance active est présente avec une granulométrie inférieure à 100, de préférence, inférieure à 50 et, en particulier, inférieure à 20 µm.

4. Composition effervescente selon l'une des revendications précédentes, **caractérisée en ce qu'**au moins une partie du sel est présente sous la forme d'un sel acide, en particulier en une quantité - calculée par rapport à la phase de substance active - allant d'environ 35 à 55 pour-cent en poids pour le citrate monosodique et / ou d'environ 40 à 65 pour-cent en poids pour le tartrate monosodique, le cas échéant présents tous deux comme un mélange de portions aliquotes.

5. Composition effervescente selon l'une des revendications précédentes, **caractérisée en ce que** l'agent liant de la phase de substance active est constitué par environ 4 à environ 13 et, de préférence, par 7 à 12 parties en poids depolyvinylpyrrolidone, pour 100 parties en poids de substance active.

6. Composition effervescente selon l'une des revendications précédentes, **caractérisée en ce que** les composantes acides de la base effervescente sont constituées par, au moins, un acide organique alimentaire solide, en mélange avec, au moins, un sel acide d'un tel acide.

7. Composition effervescente selon l'une des revendications précédentes, **caractérisée en ce que** la phase de substance active contient une partie des composantes produisant un gaz de la composition, de préférence, à raison d'environ 5 à environ 15 et, surtout, de 8 à 12 pour-cent en poids de la quantité totale des composantes produisant un gaz de la composition.

8. Composition effervescente selon l'une des revendications précédentes, **caractérisée en ce que** la phase de substance active contient d'environ 15 à environ 25 et, de préférence, de 18 à 22 pour-cent en poids d'un adjuvant neutre ou d'un excipient neutre, choisi, en particulier, parmi les substances que sont le sorbitol, le mannitol et / ou la maltodextrine.

9. Composition effervescente selon l'une des revendications précédentes, **caractérisée en ce qu'**elle est constituée, au moins, par une base effervescente et une phase de substance active, présentes en étant mutuellement séparées, ainsi que, de préférence, par une phase d'un émulsifiant et / ou par une phase d'un agent antimousse.

10. Procédé de préparation d'une composition effervescente selon l'une des revendications précédentes, **caractérisé en ce que** l'on prépare d'abord et séparément, une phase de la substance active et une base effervescente, la préparation de la phase de la substance active étant effectuée en mélangeant la substance active avec un sel, de préférence acide, d'un acide organique alimentaire, ainsi que, le cas échéant, avec, au moins, un agent liant pharmaceutiquement acceptable, et / ou, au moins, un adjuvant ou un excipient neutre et pharmaceutiquement acceptable, et / ou une partie des composantes produisant un gaz, en effectuant une granulation à l'aide d'une solution éthanolique d'un agent liant, dans laquelle on avait mis en suspension du bicarbonate de sodium utilisé comme composante produisant un gaz, en séchant et en broyant pour avoir la granulométrie souhaitée, pendant que la base effervescente, qui est constituée par un acide organique alimentaire solide, ainsi que, le cas échéant, par au moins un sel - de préférence acide - d'un tel acide, en mélange avec des carbonates ou des bicarbonates de métaux alcalins et / ou alcalino-terreux, de préférence en y incluant un édulcorant est granulée et séchée, puis broyée à la granulométrie souhaitée ; en mélangeant finalement les deux phases, en leur ajoutant, en plus et de préférence, un adjuvant ou un excipient, en particulier neutre, et en comprimant, le cas échéant pour former des comprimés.

11. Procédé selon la revendication 10, **caractérisé en ce qu'**en outre, on prépare séparément l'une de l'autre, au moins une phase d'un émulsifiant et / ou d'un agent antimousse, en ajoutant à 100 parties en poids d'un excipient, comme par exemple le mannitol ou le sorbitol, de 0,02 à 1,0 et, de préférence, environ 0,1 parties en poids d'au moins un agent antimousse et / ou d'environ 0,2 à 2,0 et, de préférence, environ 1,0 partie en poids d'au moins un émulsifiant et / ou d'un tensioactif, la phase de l'émulsifiant et / ou de l'agent antimousse ainsi préparée étant ensuite mélangée avec la base effervescente et avec la phase de la substance active.
